(19) Europäisches Patentamt
European Patent Office
Office européen des brevets

(11) Veröffentlichungsnummer: **0 226 173**
**B1**

(12) **EUROPÄISCHE PATENTSCHRIFT**

(45) Veröffentlichungstag der Patentschrift: **14.03.90**

(21) Anmeldenummer: **86117179.1**

(22) Anmeldetag: **10.12.86**

(51) Int. Cl.$^5$: **C 07 H 15/252,**
C 07 D 309/14, A 61 K 31/70,
A 61 K 31/35

(54) **Neue Anthracycline und diese enthaltende Arzneimittel.**

(30) Priorität: **17.12.85 FR 8518661**

(43) Veröffentlichungstag der Anmeldung:
**24.06.87 Patentblatt 87/26**

(45) Bekanntmachung des Hinweises auf die Patenterteilung:
**14.03.90 Patentblatt 90/11**

(84) Benannte Vertragsstaaten:
**AT BE CH DE ES GB IT LI LU NL SE**

(56) Entgegenhaltungen:
**EP-A-0 131 285**
**WO-A-86/04332**
**GB-A-2 028 336**

**JOURNAL OF MEDICINAL CHEMISTRY, Band 27, 1984, Seiten 638-645, American Chemical Society, Washington, DC, US; E.M. ACTON et al.: "Intensely potent morpholinyl anthracyclines"**

(73) Patentinhaber: **LABORATOIRES HOECHST S.A.**
**TOUR ROUSSEL HOECHST, 1 Terrasse Bellini**
**F-92800 Puteaux (FR)**
(73) Patentinhaber: **BEHRINGWERKE Aktiengesellschaft**
**Postfach 1140**
**D-3550 Marburg 1 (DE)**

(72) Erfinder: **Gesson, Jean-Pierre**
**La Germonière Montamisé**
**F-86360 Chasseneuil du Poitou (FR)**
Erfinder: **Mondon, Martine**
**9, rue de Pontreau Appt. 529**
**F-86000 Poitiers (FR)**
Erfinder: **Jacquesy, Jean-Claude**
**46, rue du Planty**
**F-86180 Bruxerolles (FR)**
Erfinder: **Kraemer, Hans Peter, Dr.**
**Birkenweg 16**
**D-3550 Marburg (DE)**

(74) Vertreter: **Orès, Bernard et al**
**Cabinet ORES 6, Avenue de Messine**
**F-75008 Paris (FR)**

EP 0 226 173 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Vorliegende Erfindung betrifft neue Anthracycline und diese enthaltende Arzneimittel.

Sehr eingehende Untersuchungen verschiedener Forschungsgruppen in den letzten Jahren erlaubten eine Erweiterung der Familie·der Anthracycline, welche heute wegen ihrer krebsbekämpfenden Wirkungen eine der vielversprechendsten Arzneimittelklassen darstellen.

Man kann insbesondere die internationale Patentanmeldung PCT no. WO 86/04332 anführen, welche offenkettige Adriamycine von Morpholintyp beschreibt.

Man kann sich ebenfalls auf den Artikel von J. Acton und al. beziehen, der in der Zeitschrift J. Med. Chem., 1984, 27, 638—645 veröffentlicht wurde und hochaktive Anthracycline betrifft.

Die angeführten Referenzen lösen nicht alle bei der Tumorbekämpfung anfallenden Probleme, insbesondere nicht das Problem der gekreutzten Widerstände.

Bei der schon über mehrere Jahre durchgeführten Untersuchung (vgl. insbesondere die französlichen Patente 83 05217, 83 13877, 84 03634, 84 09405 und 85 10063) hat die Anmelderin über neue Anthracyclinderivate berichtet, welche die pharmakologischen Eigenschaften dieser Klasse in sehr interessanter Weise abändern.

Gegenstand vorliegender Erfindung sind neue, der nachfolgenden Formel I entsprechende Anthracycline:

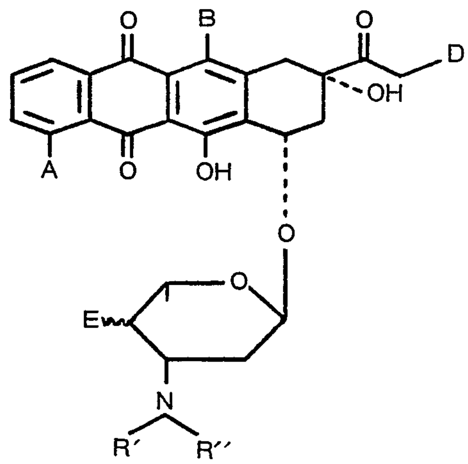

(I)

worin

A für die Gruppen $OCH_3$, OH oder H,

B für die Gruppe OH oder ein Wasserstoffatom,

D für ein Wasserstoffatom oder die Gruppe OH,

E für die Gruppe OH oder ein Wasserstoffatom und

R′ und R″, die gleich oder verschieden sein können, für ein Wasserstoffatom (wobei R′ dann von R″ verschieden ist) oder die Gruppe $-(CH_2)_n-R_1-(CH_2)_m-R_2$ stehen,

wobei

n 1 bis 6 und

m 0 bis 4 (natürlich unter der Bedingung, dass bei m = 0 $R_2$ abwesend ist) betragen und

$R_1$ eine der folgenden Gruppen:

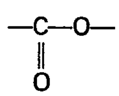

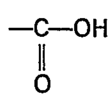

(im Fall m = o)

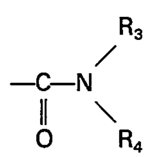

(im Fall m = 0, wobei $R_3$ und $R_4$ gleich oder verschieden sind und ein Wasserstoffatom oder eine gegebenenfalls substituierte Alkylgruppe darstellen)

—S— (vorausgesetzt, dass m von 0 verschieden ist)

—O— (vorausgesetzt, dass m von 0 verschieden ist)

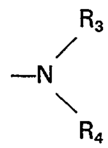

(m = 0 sowie $R_3$ und $R_4$ mit den obigen Bedeutungen)

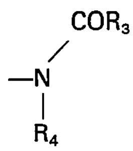

(m = 0 sowie $R_3$ und $R_4$ mit den obigen Bedeutungen)
und

$R_2$ (falls m von 0 verschieden ist) ein Wasserstoffatom oder eine Alkyl-, Alkoxy oder

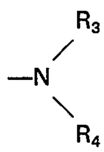 -Gruppe

(wobei $R_3$ und $R_4$ die obigen Bedeutüngen haben) bedeuten,
vorausgesetzt, dass wenn n = 1 und m = 0, $R_1$ nicht $—NR_3R_4$ oder $NR_4—COR_3$ $R_1$ ist.

Einige dieser neuen Derivate besitzen bemerkenswerte cytotoxische Eigenschaften, wie der Zellproliferations-test weiter unten zeigt. Die Anmelderin erzielte diese Ergebnisse dank der "Funktionalisierung" der Alkylreste R′ und R″.

Unter den verschiedenen erfindungsgemässen, vielversprechende pharmakologische Ergebnisse aufweisenden Derivaten seien die folgenden speziell genannt:

3′-N-(2-Methoxyäthyl)-daunorubicin,
3′-N-[(N,N-Diäthylcarbamoyl)-methyl]-daunorubicin,
3′-N,N-Bis-[(N,N-diäthylcarbamoyl)-methyl]-daunorubicin,
3′-N-(Carbomethoxymethyl)-daunorubicin,
3′-N-[2-(2-methoxyäthoxy)-äthyl]-daunorubicin und
3′-N,N-Bis-[2-(2-methoxyäthoxy)-äthyl]-daunorubicin.

Gegenstand der Erfindung sind auch Verfahren für die Herstellung der neuen Anthracycline nach Formel I und insbesondere der 4′- Epi und 4′-Desoxy-Derivate der Familie der Doxorubicine, der Daunorubicine und der Carminomycine, wobei die Verfahren dadurch charakterisiert sind, daß man in einem geeigneten Lösungsmittel, wie z. B. DMF (Dimethylformamid) und in Gegenwart eines tertiären Amins, Doxorubicin, Daunorubicin, Carminomycin ode eine entsprechende Verbindung mit einer oder mehreren Halogen-Verbindungen aus der Gruppe reagieren läßt, die insbesondere umfaßt: Halogenonitril, Halogeno-N,N-diethylacetamid, Halogenmethylacetat, 2-(2-Methoxyethoxy)-ethylhalogenid, 1-Halogen-2-methoxyethan und dann die gewünschten Produkte in geeigneter Weise abtrennt.

Dem besseren Verständnis der Erfindung dient die nachfolgende ergänzende Beschreibung, die sich mit Ausführungsbeispielen für die Verfahren und Herstellung der erfindungsgemässen Derivate sowie mit einem pharmakologischen Versuchsbericht befasst.

Es versteht sich jedoch, dass diese Ausführungsbeispiele und dieser pharmakologische Versuchsbericht ausschliesslich der Erläuterung des Erfindungsgegenstands dienen und in keiner Weise eine Einschränkung darstellen.

## Beispiele

Herstellung von 3′-N-(2-Methoxyäthyl)-daunorubicin

Ein Gemisch von Daunorubicin-chlorhydrat (30 mg; 0,054 mmol) und $Et_3N$ (0,16 mmol) in 1,5 ml DMF versetzt man mit 2-Jodäthylmethyläther (15 val). Nach 48 Stunden bei gewöhnlicher Temperatur trennt man das 3′-N-(2-Methoxyäthyl)-daunorubicin (60%) vom verbleibenen Daunorubicin (22%).

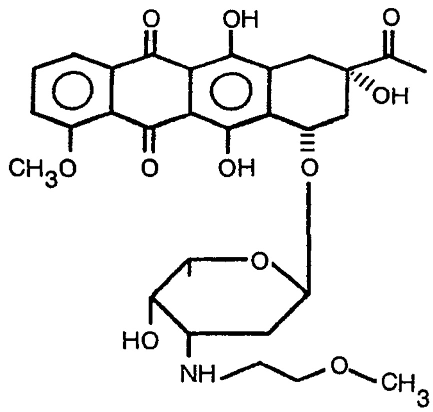

3'-N-(2-Methoxyäthyl)-daunorubicin

Ausbeute: 60%
Schmelzpunkt: (Koflerbank): 113°C (Zers.)
Massenspektroskopie: (FAB): 585 (12%); 515 (13%); 409 (9%); 380 (11%); 321 (12%); 301 (100%)
$[\alpha]_D$ = 253° (c = 0,15; $CHCl_3$)

NMR ($CDCl_3$) 200 MHz: 1,38 (Dublett, J=5.5Hz, 3H); 2,41 (Singlett, 3H); 2,74 (Triplett, J=5Hz, 2H); 3,32 (Singlett, 3H); 3,46 (Triplett, J=5Hz, 2H); 4,09 (Singlett, 3H); 4,73 (breites Singlett, 1H); 5,31 (scharfes Signal, 1H); 5,53 (scharfes Signal, 1H); 7.40 (Dublett, J=7,5 Hz, 1H); 7,80 (Triplett, J=7,5Hz, 1H); 8,06 (Dublett, J=7,5Hz, 1H).

Auf die gleiche Weise erhält man:

a)

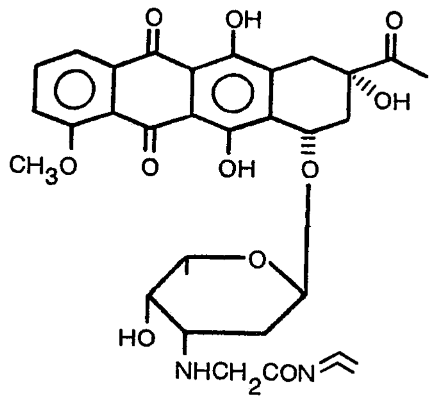

3'-N-[(N,N-Diäthylcarbamoyl)-methyl]-daunorubicin

NMR: 1,37 (Dublett, J=5,5Hz, 3H); 2,41 (Singlett, 3H); 4,08 (Singlett, 3H); 4,77 (breites Singlett, 1H); 5,31 (scharfes Signal, 1H); 5,53 (scharfes Signal, 1H); 7,39 (Dublett, J=7,5Hz, 1H); 7,78 Triplett, J=7,5Hz, 1H); 8,03 (Dublett, J=7,5Hz, 1H); 13,30 (Singlett, 1H); 13,66 (Singlett, 1H).

b)

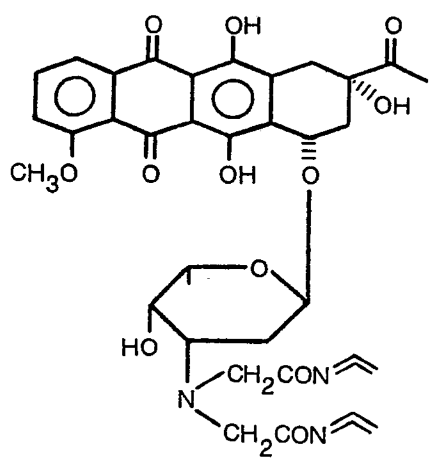

3'-N-Bis-[(N,N-diäthylcarbamoyl)-methyl]-daunorubicin

NMR: 2,40 (Singlett, 3H); 4,09 (Singlett, 3H); 5,30 (scharfes Signal, 1H); 5,57 (scharfes Signal, 1H); 7,38 (Dublett, J=7,5Hz, 1H); 7,77 (Triplett, J=7,5Hz, 1H); 8,05 (Dublett, J=7,5Hz, 1H); 13,31 (Singlett, 1H); 13,65 (Singlett, 1H).

c)

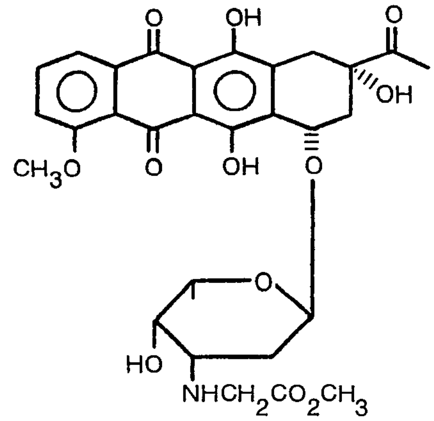

3′-N-(Carbomethoxymethyl)-daunorubicin

Schmelzpunkt: 142—148°C

NMR ($CDCl_3$) 200 MHz: 1,36 (Dublett, J=5,5Hz, 3H); 2,41 (Singlett, 3H); 3,10 (ABq, 2H); 3,41 (Singlett, 2H); 3,52 (Singlett, 1H); 3,70 (Singlett, 3H); 4,09 (Singlett, 3H); 5,29 (scharfes Signal, 1H); 5,52 (scharfes Signal, 1H); 7,40 (Dublett, J=7,5Hz, 1H); 7,79 (Triplett, J=7,5Hz, 1H); 8,06 (Dublett, J=7,5Hz, 1H).

d)

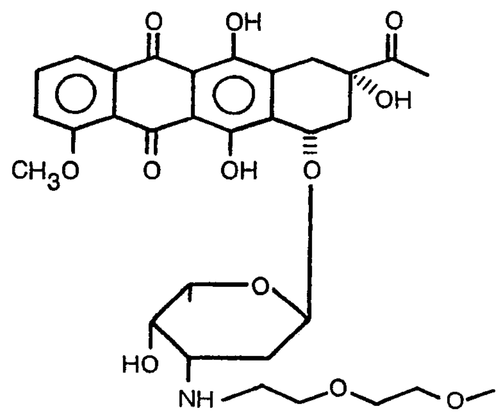

3′-N-[2-(2-Methoxyäthoxy)-äthyl]-daunorubicin

Ausbeute = 44%

Schmelzpunkt: 70—75°C

$[\alpha]_D$ = 270° (c = 0,1; $CHCl_3$)

NMR ($CDCl_3$) 200 MHz: 1,36 (Dublett, J=5,5Hz, 3H); 2,41 (singlett, 3H); 3,02 (ABq, 2H); 3,33 (Singlett, 3H); 4,07 (Singlett, 3H); 4,69 (breites Singlett, 1H); 5,25 (scharfes Signal, 1H); 5,50 (scharfes Signal, 1H); 7,38 (Dublett, J=5Hz, 1H); 7,76 (Triplett, J=7,5Hz, 1H); 8,00 (Dublett, J=7,5Hz, 1H).

e)

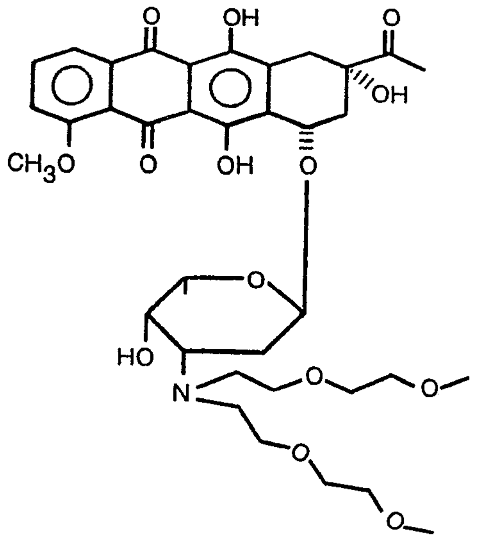

3'-N,N-Bis-[2-(2-Methoxyäthoxy)-äthyl]-daunorubicin

Ausbeute = 9%
$[\alpha]_D$ = 294° (c = 0,07; $CHCl_3$)
NMR ($CDCl_3$) 200 MHz: 1,34 (Dublett, J=5Hz, 3H); 2,42 (Singlett, 3H); 3,12 (ABq, 2H); 3,31 (Singlett, 6H); 4,09 (Singlett, 3H); 4,74 (breites Singlett, 1H); 5,30 (scharfes Signal, 1H); 5,58 (scharfes Signal, 1H); 7,40 (Dublett, J=7,5Hz, 1H); 7,79 (Triplett, J=7,5Hz, 1H); 8,02 (Dublett, J=7,5Hz, 1H); 13,31 (Singlett, 1H); 14,0 (Singlett, 1H).

Die cytotoxischen Eigenschaften gewisser erfindungsgemäss beanspruchter Derivate werden durch die in der nachfolgenden Tabelle I zusammengestellten Zellproliferations-tests nachgewiesen.

Der Versuchsplan wurde gemäss der Arbeitsweise nach Hamburger und Salmon an Leukämiezellen des Stammes L 1210 durchgeführt. Dabei wurden gewisse Abänderungen an der Arbeitsweise nach Hamburger und Salmon vorgenommen, insbesondere:

Das konditionierte Medium wurde durch McCoy5A ersetzt. Die Anzahl Zellen pro Schale wurde wegen der hohen Waschstumsgeschwindigkeit der Leukämizellen L 1210 auf 5 × $10^2$ Zellen pro Schale verringert.

Zellen wurden mit verschiedenen Konzentrationen der Versuchssubstanz 1 Stunde lang bei 37°C inkubiert. Dann wurden die Zellen zweimal mit McCoy5A gewaschen und schliesslich gemäss der Methode nach Hamburger und Salmon in einem Zweischichtenager ausplattiert.

Ferner wurden Parallelversuche mit kontinuierlicher Inkubation und Zugabe der Versuchssubstanz in verschiedenen Konzentrationen in die obere Agarschicht vor der Plattierung durchgeführt.

Die Schalten wurden 5 bis 7 Tage lang bei 37°C unter 5% $CO_2$, 20% $O_2$ und 95% relativer Feuchte in einem Inkubator gelagert. Nach diesem Zeitraum wurden die Kolonien mit einem Durchmesser über 60 µm in einem Invertoskop gezählt.

Die Ergebnisse sind als Prozentsatz der Anzahl aus L 1210 gebildeter, behandelter Kolonien gegenüber der Koloniezahl einer unbehandelten Kontrolle ausgedrückt. Die Standardabweichung bei Wiederholungsversuchen betrug weniger als 15%.

Die Ergebnisse sind in Tabelle I angegeben.

TABELLE I

| Produkt | Hemmkonzentration $IC_{50}$ in µg/ml (kontinuierliche Exposition) |
|---|---|
| 3'-N-(2-Methoxyäthyl)-daunorubicin | 0,01 |
| 3'-N-[(N,N-Diäthylcarbamoyl)-methyl]-daunorubicin | 0,28 |
| 3'-N,N-Bis[(N,N-diäthylcarbamoyl)-methyl]-daunorubicin | 0,65 |
| 3'-N-(Carbomethoxymethyl)-daunorubicin | 0,24 |
| 3'-N-[2-(2-Methoxyäthoxy)-äthyl]-daunorubMethoxyäthoxy)-äthyl]-daunorubicin | 0,07 |
| 3'-N,N-Bis-[2-(2-methoxyäthoxy)-äthyl]-daunorubicin | 0,22 |
| Doxorubicin (Kontrolle) | 0,02 |
| Daunorubicin (Kontrolle) | 0,02 |

Die erfindungsgemässe Derivate enthaltenden Arzneimittel werden im allgemeinen in Dosierungen zwischen 0,001 und 25 mg/kg/Tag verabreicht. Vorzugsweise erfolgt die Verabreichung der Derivate der allgemeinen Formel I auf oralem Wege, doch können sie nach Bedarf auf anderen Wegen verabreicht werden: parenteral, subkutan, intravenös, intramuskulär, intraperitoneal oder transkutan.

Diese Verabreichung kann ebenfalls kombiniert mit pharmazeutisch unbedenklichen Trägerstoffen oder Streckmitteln erfolgen.

**Patentansprüche für die Vertragsstaaten: BE CH DE GB IT LI LU NL SE**

1. Neue Anthracycline der folgenden Formel I:

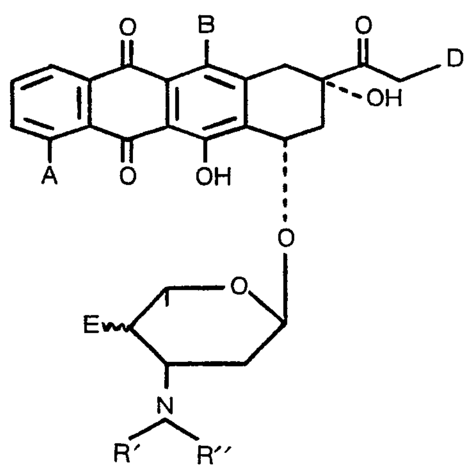

(I)

worin

A für die Gruppen $OCH_3$, OH oder H,

B für die Gruppe OH oder ein Wasserstoffatom,

D für ein Wasserstoffatom oder die Gruppe OH,

E für die Gruppe OH oder ein Wasserstoffatom und

R' und R'', die gleich oder verschieden sein können, für ein Wasserstoffatom (wobei R' dann von R'' verschieden ist) oder die Gruppe $-(CH_2)_n-R_1-(CH_2)_m-R_2$ stehen,

wobei

n 1 bis 6 und

m 0 bis 4 (natürlich unter der Bedingung, dass bei m = 0 $R_2$ abwesend ist) betragen und

$R_1$ eine der folgenden Gruppen:

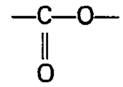

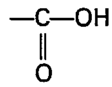

(im Fall m = 0)

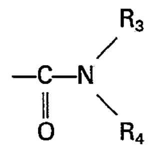

(im Fall m = 0, wobei $R_3$ und $R_4$ gleich oder verschieden sind und ein Wasserstoffatom oder eine gegebenenfalls substituierte Alkylgruppe darstellen)

—S— (vorausgesetzt, dass m von 0 verschieden ist)

—O— (vorausgesetzt, dass m von 0 verschieden ist)

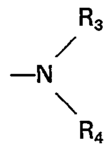

(m = 0 sowie $R_3$ und $R_4$ mit den obigen Bedeutungen)

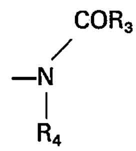

(m = 0 sowie $R_3$ und $R_4$ mit den obigen Bedeutungen)
und

$R_2$ (falls m von 0 verschieden ist) ein Wasserstoffatom oder eine Alkyl-, Alkoxy oder

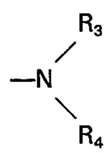 -Gruppe

(wobei $R_3$ und $R_4$ die obigen Bedeutüngen haben) bedeuten,
vorausgesetzt, dass wenn n = 1 und m = 0, $R_1$ nicht —$NR_3R_4$ oder $NR_4$—$COR_3$ $R_1$ ist.

2. Anthracycline nach Anspruch 1, dadurch gekennzeichnet, dass sie Derivate aus der Klasse des Doxorubicins darstellen.

3. Anthracycline nach Anspruch 1, dadurch gekennzeichnet, dass sie Derivate aus der Klasse des Daunorubicins darstellen.

4. Anthracycline nach Anspruch 1, dadurch gekennzeichnet, dass sie Derivate aus der Klasse des Carminomycins darstellen.

5. Anthracycline nach Anspruch 1, dadurch gekennzeichnet, dass diese Epi-4'-Derivate sind.

6. Anthracycline nach Anspruch 1, dadurch gekennzeichnet, dass diese Desoxy-4'-Derivate sind.

7. 3'-N-(2-Methoxyäthyl)-daunorubicin als Anthracyclin nach Anspruch 1.

8. 3'-N-[(N,N-Diäthylcarbamoyl)-methyl]-daunorubicin als Anthracyclin nach Anspruch 1.

9. 3'-N,N-Bis[(N,N-diäthylcarbamoyl)-methyl]-daunorubicin als Anthracyclin nach Anspruch 1.

10. 3'-N-(Carbomethoxymethyl)-daunorubicin als Anthracyclin nach Anspruch 1.

11. 3'-N-[2-(2-Methoxyäthoxy)-äthyl]-daunorubicin als Anthracyclin nach Anspruch 1.

12. 3'-N,N-Bis-[2-(2-methoxyäthoxy)-äthyl]-daunorubicin als Anthracyclin nach Anspruch 1.

13. Aus Anthracyclinen nach einem der Ansprüche 1 bis 12 bestehende oder diese enthaltende Arzneimittel.

14. Verfahren zur Herstellung der Anthracycline nach Anspruch 1, dadurch gekennzeichnet, daß man in einem geeigneten Lösungsmittel wie DMF und in Gegenwart eines tertiären Amins Doxorubicin,

Daunorubicin, Carminomycin oder eine entsprechende Verbindung zur Reaktion bringt mit einer oder mehreren Halogenverbindungen aus der Gruppe der Verbindungen, die insbesondere umfaßt Halogenonitril, Halogeno-N,N-Diethylacetamid, Halogenmethylacetat, 2-(2-Methoxyethoxy)-ethylhalogenid, 1-Halogen-2-methoxyethan, und daraufhin das gewünschte Produkt in geeigneter Weise abtrennt.

**Patentansprüche für die Vertragsstaaten: AU ES**

1. Verfahren zur Herstellung neuer Anthracycline der folgenden Formel I:

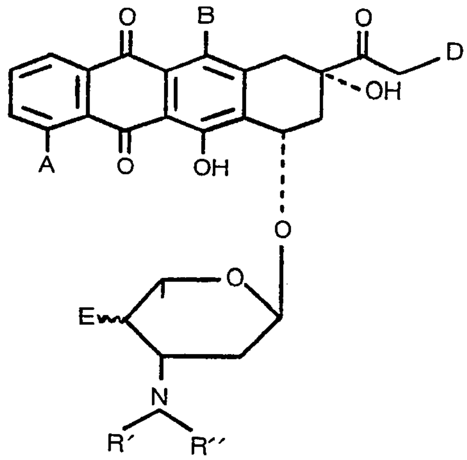

(I)

worin

A für die Gruppen $OCH_3$, OH oder H,

B für die Gruppe OH oder ein Wasserstoffatom,

D für ein Wasserstoffatom oder die Gruppe OH,

E für die Gruppe OH oder ein Wasserstoffatom und

R′ und R″, die gleich oder verschieden sein können, für ein Wasserstoffatom (wobei R′ dann von R″ verschieden ist) oder die Gruppe $-(CH_2)_n-R_1-(CH_2)_m-R_2$ stehen,

wobei

n 1 bis 6 und

m 0 bis 4 (natürlich unter der Bedingung, dass bei m = 0 $R_2$ abwesend ist) betragen und

$R_1$ eine der folgenden Gruppe:

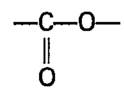

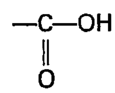

(im Fall m = 0)

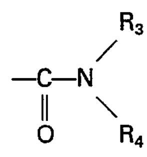

(im Fall m = 0, wobei $R_3$ und $R_4$ gleich oder verschieden sind und ein Wasserstoffatom oder eine gegebenenfalls substituierte Alkylgruppe darstellen)

—S— (vorausgesetzt, dass m von 0 verschieden ist)

—O— (vorausgesetzt, dass m von 0 verschieden ist)

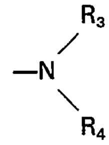

(m = 0 sowie $R_3$ und $R_4$ mit den obigen Bedeutungen)

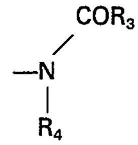

(m = 0 sowie $R_3$ und $R_4$ mit den obigen Bedeutungen)
und

$R_2$ (falls m von 0 verschieden ist) ein Wasserstoffatom oder eine Alkyl-, Alkoxy oder

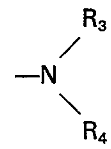 -Gruppe

(wobei $R_3$ und $R_4$ die obigen Bedeutüngen haben) bedeuten,
vorausgesetzt, dass wenn n = 1 und m = 0, $R_1$ nicht $—NR_3R_4$ oder $NR_4—COR_3$ $R_1$ ist.

Und insbesondere zur Herstellung der 4'-Epi- und der 4'-Desoxy-Derivate der Familie der Doxorubicine, der Daunorubicine und der Carminomycine, dadurch gekennzehchnet, daß man in einem geeigneten Lösungsmittel wie DMF und in Gegenwart eines tertiären Amins Doxorubicin, Daunorubicin, Carcinomycin oder eine entsprechende Verbindung zur Reaktion bringt mit einer oder mehreren Halogenverbindungen aus der Gruppe der Verbindungen, die insbesondere umfaßt Halogennitrile, Halogeno-N,N-diethylacetamid, Halogenmethylacetat, 2-(2-Methoxyethoxy)-ethylhalogenid, 1-Halogen-2-methoxyethan, und daraufhin das gewünschte Produkt in geeigneter Weise abtrennt.

2. Verfahren nach Anspruch 1, dadurch gekennzeichnet, daß Produkte aus der Gruppe von Verbindungen erhalten werden, die besteht aus

3'-N-(2-Methoxyäthyl)-daunorubicin, 3'-N-(N,N-Diäthylcarbamoyl)-methyl-daunorubicin, 3'-N,N-Bis-(N,N-diäthylcarbamoyl)-methyl-daunorubicin, 3'-N-(Carbomethoxymethyl)-daunorubicin, 3'-N-2-(2-Methoxyäthoxy)-äthyl-daunorubicin und 3'-N,N-Bis-2-(2-methoxyäthoxy)-äthyl-daunorubicin.

**Revendications pour les Etats contractants: BE CH DE GB IT LI LU NL SE**

1. Nouvelles anthracyclines représentées par la formule I ci-après:

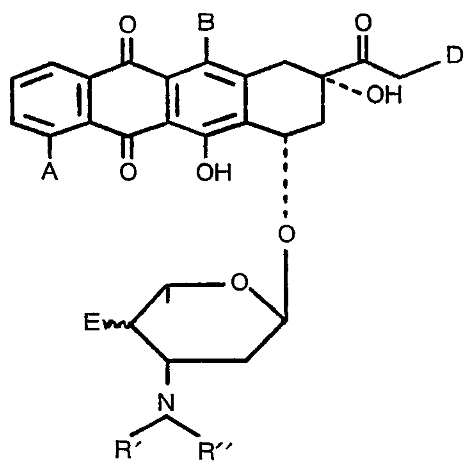

(I)

dans laquelle:

A représente les groupes $OCH_3$ ou OH ou H,

B représente le groupe OH ou un atome d'hydrogène,

D représente un atome d'hydrogène ou le groupe OH,

E représente le groupe OH ou un atome d'hydrogène,

et R' et R'' qui peuvent être identiques ou différents représentent un atome d'hydrogène (et dans ce cas R' est différent de R'') ou le groupe $—(CH_2)_n—R_1—(CH_2)_m—R_2$
où

n est compris entre 1 et 6,

m est compris entre 0 et 4 (avec, bien entendu, la condition que si m = 0, $R_2$ est nul),

$R_1$ représente un des groupes suivants:

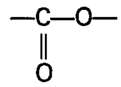

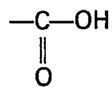

(dans ce cas m = 0)

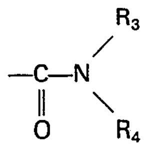

(dans ce cas m = 0, où $R_3$ et $R_4$ identiques ou différents représentent un atome d'hydrogène ou un groupe alkyle substitué ou non)

—S— (avec la condition que m soit différent de 0)

—O— (avec la condition que m soit différent de 0)

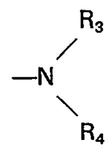

(m = 0 et $R_3$ et $R_4$ ont la même signification que ci-dessus)

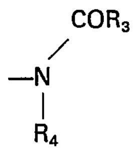

(m = 0 et $R_3$ et $R_4$ ont la même signification que ci-dessus)

et

$R_2$ représente (dans le cas où m est différent de 0) un atome d'hydrogène ou les groupes alkyle, alcoxy ou

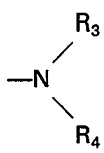

($R_3$ et $R_4$ ayant la même signification que ci-dessus),

à la condition que, lorsque n = 1 et m = 0, $R_1$ ne soit ni un groupe $—NR_3R_4$, ni un groupe $NR_4—COR_3$.

2. Anthracyclines selon la revendication 1, caractérisées en ce que ce sont des dérivés de la famille de la doxorubicine.

3. Anthracyclines selon la revendication 1, caractérisées en ce que ce sont des dérivés de la famille de la daunorubicine.

4. Anthracyclines selon la revendication 1, caractérisées en ce que ce sont des dérivés de la famille de la carminomycine.

5. Anthracyclines selon la revendication 1, caractérisées en ce que ce sont des dérivés épi-4'.

6. Anthracyclines selon la revendication 1, caractérisées en ce que ce sont des dérivés désoxy-4'.

7. Anthracycline selon la revendication 1, caractérisée en ce qu'elle est constituée par la N-(méthoxy-2 éthyl)-3' daunorubicine.

8. Anthracycline selon la revendication 1, caractérisée en ce qu'elle est constituée par la N-(N,N-diéthylcarbamoyl)méthyl-3' daunorubicine.

9. Anthracycline selon la revendication 1, caractérisée en ce qu'elle est constituée par la N,N-bis-(N,N-diéthylcarbamoyl)méthyl-3' daunorubicine.

10. Anthracycline selon la revendication 1, caractérisée en ce qu'elle est constituée par la N-(carbométhoxyméthyl)-3' daunorubicine.

11. Anthracycline selon la revendication 1, caractérisée en ce qu'elle est constituée par la N-[(méthoxy-2 éthoxy)-2 éthyl]-3' daunorubicine.

12. Anthracycline selon la revendication 1, caractérisée en ce qu'elle est constituée par la N,N-bis-[(méthoxy-2 éthoxy)-2 éthyl]-3' daunorubicine.

13. Médicaments constitués ou contenant des anthracyclines selon l'une quelconque des revendications 1 à 12.

14. Procédé de préparation des anthracyclinones selon la revendication 1, caractérisé en ce que l'on fait réagir dans un solvant approprié, tel que le DMF et en présence d'une amine tertiaire, la doxorubicine ou la daunorubicine ou la carminomycine ou un de leur sel correspondant avec un ou plusieurs dérivés halogénés pris dans le groupe qui comprend notamment l'halogèno-nitrile, l'halogèno-N,N diéthylacétamide, l'halogèno-acétate de méthyle, l'halogèno-1(méthoxy-2 éthoxy)-2 éthane, l'halogèno-1 méthoxy-2 éthane, et en ce que l'on isole le produit désire de manière appropriée.

**Revendications pour les Etats contractants: AT ES**

1. Procédé pour la préparation de nouvelles anthracyclines représentées par la formule I ci-après:

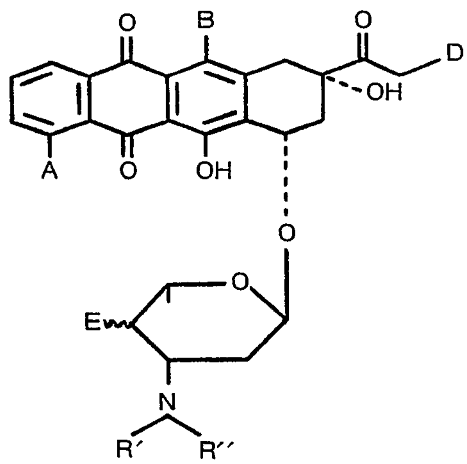

(I)

dans laquelle:

A représente les groupes $OCH_3$ ou OH ou H,
B représente le groupe OH ou un atome d'hydrogène,
D représente un atome d'hydrogène ou le groupe OH,
E représente le groupe OH ou un atome d'hydrogène,

et R' et R'' qui peuvent être identiques ou différents représentent un atome d'hydrogène (et dans ce cas R' est différent de R'') ou le groupe $-(CH_2)_n-R_1-(CH_2)_m-R_2$
où

n est compris entre 1 et 6,
m est compris entre 0 et 4 (avec, bien entendu, la condition que si m = 0, $R_2$ est nul),
$R_1$ représente un des groupes suivants:

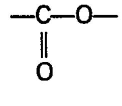

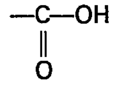

(dans ce cas m = 0)

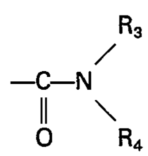

(dans ce cas m = 0, où $R_3$ et $R_4$ identiques ou différents représentent un atome d'hydrogène ou un groupe alkyle substitué ou non)

—S— (avec la condition que m soit différent de 0)
—O— (avec la condition que m soit différent de 0)

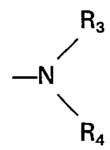

(m = 0 et $R_3$ et $R_4$ ont la même signification que ci-dessus)

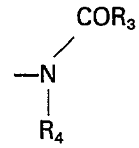

(m = 0 et $R_3$ et $R_4$ ont la même signification que ci-dessus)
et

$R_2$ représente (dans le cas où m est différent de 0) un atome d'hydrogène ou les groupes alkyle, alcoxy ou

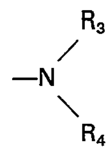

($R_3$ et $R_4$ ayant la même signification que ci-dessus),
à la condition que, lorsque n = 1 et m = 0, $R_1$ ne soit ni un groupe $—NR_3R_4$, ni un groupe $NR_4—COR_3$, et notamment de dérivés épi-4' et désoxy-4' de la famille de la doxorubicine, de la famille de la daunorubicine et de la famille de la carminomycine, caractérisé en ce que l'on fait réagir dans un solvant approprié, tel que le DMF et en présence d'une amine tertiaire, la doxorubicine ou la daunorubicine ou la carminomycine ou un de leur sel correspondant avec un ou plusieurs dérivés halogénés pris dans le groupe qui comprend notamment l'halogèno-nitrile, l'halogèno-N,N diéthylacétamide, l'halogèno-acétate de méthyle, l'halogèno-1(méthoxy-2 éthoxy)-2 éthane, l'halogèno-1 méthoxy-2 éthane, et en ce que l'on isole le produit désiré de manière appropriée.

2. Procédé selon la revendication 1, caractérisé en ce que les composés obtenus sont constitués par:
— la N-(méthoxy-2 éthyl)-3' daunorubicine,
— la N-(N,N-diéthylcarbamoyl)méthyl-3' daunorubicine,
— la N,N-bis-(N,N-diéthylcarbamoyl)méthyl-3' daunorubicine,
— la N-(carbométhoxyméthyl)-3' daunorubicine,
— la N-[(méthoxy-2 éthoxy)-2 éthyl]-3' daunorubicine, et
— la N,N-bis-[(méthoxy-2 éthoxy)-2 éthyl)]-3' daunorubicine.

**Claims for the Contracting States: BE CH DE GB IT LI LU NL SE**

1. New anthracyclines of the following Formula I:

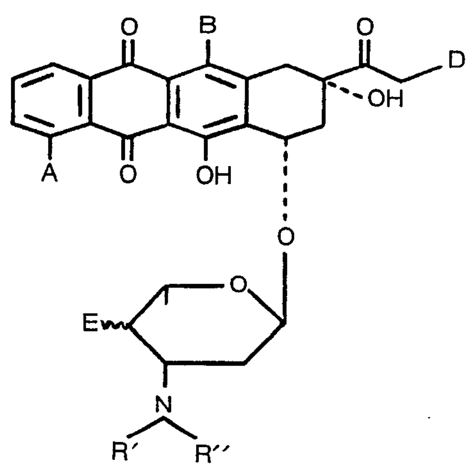

(I)

in which

A stands for the groups $OCH_3$, OH or H,

B stands for the group OH or a hydrogen atom,

D stands for a hydrogen atom or the group OH,

E stands for the group OH or a hydrogen atom and R′ and R″, which may be identical or different, stand for a hydrogen atom (in which R′ is then different from R″) or the group $-(CH_2)_n-R_1-(CH_2)_m-R_2$ in which

n is 1 to 6 and

m is 0 to 4 (naturally under the condition that with m = 0 $R_2$ is absent) and

$R_1$ denotes one of the following groups:

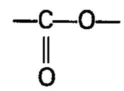

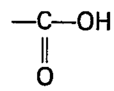

(if m = 0)

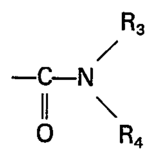

(if m = 0, in which $R_3$ and $R_4$ are identical or different and represent a hydrogen atom or an alkyl group which is substituted if required)

—S— (provided that m is different from 0)

—O— (provided that m is different from 0)

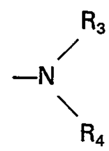

(m = 0 and $R_3$ and $R_4$ with the above meanings)

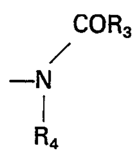

(m = 0 and $R_3$ and $R_4$ with the above meanings)
and

$R_2$ (if m is different from 0) denotes a hydrogen atom or an alkyl-, alkoxy- or

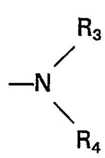 -group

(in which $R_3$ and $R_4$ have the above meanings),
provided that if n = 1 and m = 0, $R_1$ is not $-NR_3R_4$ or $NR_4-COR_3$.

2. Anthracyclines according to Claim 1, characterized in that they are derivatives from the class of doxorubicines.

3. Anthracyclines according to Claim 1, characterized in that they are derivatives from the class of daunorubicines.

4. Anthracyclines according to Claim 1, characterized in that they are derivatives from the class of carminomycines.

5. Anthracyclines according to Claim 1, characterized in that these are Epi-4′-derivatives.

6. Anthracyclines according to Claim 1, characterized in that these are desoxy-4′-derivatives.

7. 3′-N-(2-methoxyethyl)-daunorubicine as anthracycline according to Claim 1.

8. 3'-N-[N,N-diethylcarbamoyl)-methyl]-daunorubicine as anthracycline according to Claim 1.

9. 3'-N,N-bis[(N,N-diethylcarbamoyl)-methyl]-daunorubicine as anthracycline according to Claim 1.

10. 3'-N-(carbomethoxymethyl)-daunorubicine as anthracycline according to Claim 1.

11. 3'-N-[2-(2-methoxyethoxy)-ethyl]-daunorubicine as anthracycline according to Claim 1.

12. 3'-N,N-bis-[2-(2-methoxyethoxy)-ethyl]-daunorubicine as anthracycline according to Claim 1.

13. Medicaments consisting of anthracyclines according to one of Claims 1 to 12 or containing these.

14. Method for the production of the anthracyclines according to Claim 1, characterized in that in a suitable solvent such as DMF and in the presence of a tertiary amine, doxorubicine, daunorubicine, carminomycine or a corresponding compound are brought to reaction with one or more halogen compounds from the group of compounds which comprises in particular halogenonitrile, halogeno-N,N-diethylacetamide, halogen methyl acetate, 2-(2-methoxyethoxy)ethylhalogenide, 1-halogen-2-methoxyethane, and then the desired product is separated in a suitable manner.

**Claims for the Contracting States: AT ES**

1. Method for the production of new anthracyclines of the following Formula I:

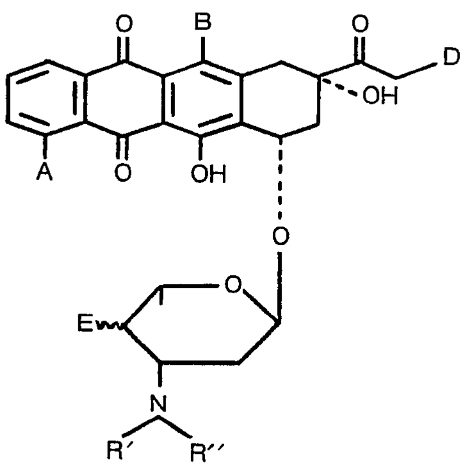

(I)

in which

A stands for the groups $OCH_3$, OH or H,

B stands for the group OH or a hydrogen atom,

D stands for a hydrogen atom or the group OH,

E stands for the group OH or a hydrogen atom and R' and R'', which may be identical or different, stand for a hydrogen atom (in which R' is then different from R'') or the group $-(CH_2)_n-R_1-(CH_2)_m-R_2$ in which

n is 1 to 6 and

m is 0 to 4 (naturally under the condition that with m = 0 $R_2$ is absent) and

$R_1$ denotes one of the following groups:

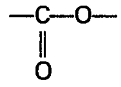

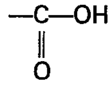

(if m = 0)

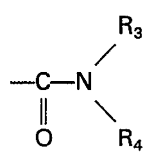

(if m = 0, in which $R_3$ and $R_4$ are identical or different and represent a hydrogen atom or an alkyl group which is substituted if required)

—S— (provided that m is different from 0)

—O— (provided that m is different from 0)

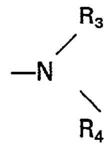

(m = 0 and $R_3$ and $R_4$ with the above meanings)

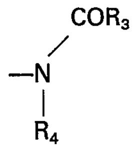

(m = 0 and $R_3$ and $R_4$ with the above meanings)
and

$R_2$ (if m is different from 0) denotes a hydrogen atom or an alkyl-, alkoxy- or

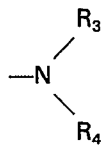 -group

(in which $R_3$ and $R_4$ have the above meanings),
provided that if n = 1 and m = 0, $R_1$ is not $—NR_3R_4$ or $NR_4—COR_3$.
and in particular for the production of the 4'-epi- and the 4'-desoxy- derivatives of the family of doxorubicines, daunorubicines and carminomycines, characterized in that in a suitable solvent such as DMF and in the presence of a tertiary amine, doxorubicine, daunorubicine, carminomycine or a corresponding compound are brought to reaction with one or more halogen compounds from the group of compounds which comprises in particular halogenonitrile, halogeno-N,N-diethylacetamide, halogen methyl acetate, 2-(2-methoxyethoxy)-ethylhalogenide, 1-halogen-2-methoxyethane, and then the desired product is separated in a suitable manner.

2. Method according to Claim 1, characterized in that products are obtained from the group of compounds which consists of

3'-N-2-methoxyethyl)-daunorubicine, 3'-N-(N,N-diethylcarbamoyl)-methyl-daunorubicine, 3'-N,N-bis-(N,N-diethylcarbamoyl)-methyl-daunorubicine, 3'-N-(carbomethoxymethyl)-daunorubicine, 3'-N-2-(2-methoxyethoxy)-ethyl-daunorubicine and 3'-N,N-bis-2-(2-methoxyethoxy)-ethyl-daunorubicine.